# EUROPEAN PATENT SPECIFICATION

(11) **EP 1 915 301 B1**
(45) Date of publication and mention of the grant of the patent: **12.08.2009**
(21) Application number: 06763586.2
(22) Date of filing: 08.06.2006
(51) Int. Cl.: B65D 83/14, G06K 19/07, A61M 15/00

(54) **AEROSOL CONTAINER**
AEROSOLBEHÄLTER
CONTENANT AEROSOL

(30) Priority: 08.06.2005 DE 102005026564
(43) Date of publication of application: 30.04.2008
(73) Proprietor: Nycomed GmbH, 78467 Konstanz (DE)
(72) Inventor: DIETRICH, Rango, 78465 Konstanz (DE); EMSER, Gabriele, 8280 Kreuzlingen (CH); PFEIFFER, Jutta, 88085 Langenargen (DE); LANGE, Michael, 78464 Konstanz (DE)
(74) Representative: Wild, Robert
(86) International application number: PCT/EP2006/063014
(87) International publication number: WO 2006/131545

(56) References cited:
- WO-A-01/62322
- US-A1- 2003 095 253
- US-A1- 2003 183 226
- US-A1- 2005 252 930

## Description

### Technical field of the invention

The invention relates to a metallic aerosol container with an RFID unit.

### Prior art

Radiofrequency identification (RFID) is a technology affording new possibilities of automation in many fields of application, including in the pharmaceutical industry, for example in the logistics involved in carrying out clinical trials. The transponders used in RFID technology are usually composed of a microchip for storing data, and of an antenna for transmitting data.

GB 2342203 describes a method for drug packaging, for use in carrying out clinical trials. Containers are provided which, for example, have an RFID tag and are filled with a specific drug that is to be packaged. The identity of the container and the identity of the drug located in the latter are stored in a database, together with data for inscriptions, and a tag containing the information stored in the database is applied to the container. The risk of applying the wrong tag is thereby avoided.

WO 01/94016 describes a sample container comprising a holder for receiving a sample, and an RFID unit (radiofrequency identifier) comprising an antenna for transmitting or receiving radiofrequencies, and an integrated circuit chip connected to the antenna, the RFID unit being arranged on a carrier, and the carrier being connected to the holder for receiving the sample. The holder is intended in particular for use in clinical trials.

WO 01/62322 is related to a medicament dispenser comprising a housing, medicament container, a dispensing mechanism for dispensing medicament from the medicament container and a radiofrequency identifier. The radiofrequency identifier comprises an antenna for transmitting or receiving radiofrequency energy and an integrated circuit chip connection with said antenna. The radiofrequency identifer connects to said housing or said medicament container.

US 2003/0183226 is related to a medicament dispenser for use in the storage, presentation and/or dispensing of medicament comprising a body shaped for receipt of a medicament container. In association with the body, there is a first transceiver for transmitting and receiving data. A medicament container is receivable by the body. In association with the medicament container there is a second transceiver for transmitting and receiving data. Data is transferable in two-way fashion from the first transceiver to the second transceiver. The medicament dispenser may be supplied in kit of parts form.

EP 1083519 is related to a method of mounting radiofrequency transponders on containers.

US 2003/0109068 is related to a fluid product distribution device which may comprise an identification unit from the radio-frequency type.

US 2001/0054755 is related to an RFID tag system for a package comprising an RFID tag having an antenna. The RFID tag is coupled to the package. An RFID antenna, is capacitively coupled to a conductive package or content material wherein said conductive package or content material forms a part of an RF antenna design.

US 2003/0095253 is related to a variety of improvements in the technology relating to containers and more particularly to the use of a mechnism in conjunction with a bottle, to determine if it has been opened.

WO 2004/049237 is related to products having RFID tags to provide information to product consumers.

Many active substances for treatment of diseases of the airways, such as asthma, are provided as preparations in pressurized containers from which they are administered into the lungs. Such a pharmaceutical product is made up, for example, of an aerosol container (pressurized container) with a dispensing valve, and of a mouthpiece in which the container is received. The valve stem of the dispensing valve is received in a nozzle section of the mouthpiece. Problems in the use of RFID tags arise in particular when the RFID unit is applied in the direct vicinity of materials such as metal and liquids, for example when the RFID tag is to be applied to the metallic pressurized container. Communication with the RFID unit is disrupted here. However, for the automation of clinical trials in which active substances in pressurized containers are used, it is desirable to be able to use RFID technology. In particular it has been proven to be difficult to carry out bulk-reading of metallic objects carrying the RFID units. Usually bulk reading allows the reading and identification of 50 to 150 RFID units per second at the same time.

### Disclosure of the invention

Surprisingly, it has now been found that, by applying the RFID unit to a spacer made of insulating material which is applied to the base of the pressurized container, a disruptive effect of the metallic pressurized container on the RFID unit can be avoided. The RFID unit is preferably placed on the outer circumference of the spacer, particularly in parallel orientation to the metallic surface of the container, which strengthens the antenna action.

Furthermore, the spacer permits "blinding" of the base of the aerosol container, i.e. printed details pointing to the nature of the product or to the manufacturer are here no longer possible.

The subject of the invention is therefore a metallic aerosol container (pressurized container) with a dispensing valve, in which a spacer made of an insulating material is applied to the base of the aerosol container and is equipped with an RFID unit.

The metallic aerosol container is preferably a pressurized container with a dispensing valve on the face directed away from the base (i.e. on the opposite side of the base of the container). The aerosol container is preferably suitable for being received in a mouthpiece, the valve stem of the dispensing valve being able to be received in a nozzle section of the mouthpiece. Examples of metals from which the aerosol container can be produced are aluminium or stainless steel.

According to the invention, the spacer is a spacer made from insulating material. Preferably the specer is made from plastics and most preferably is a plastic cylinder. The spacer is preferably configured such that it can be applied with a form fit onto the base of the aerosol container.

According to the invention, the RFID unit (according to the invention also designated as transponder) comprises a microchip for holding data, and an antenna for receiving or transmitting data. The microchip is connected to the antenna, and both can be applied on a common carrier. Moreover, an energy source can also form a component part of the RFID unit. The antenna is suitable for transmitting or receiving radiofrequency energy in the range of from 50 KHz to 2.5 GHz, in particular 13.56 MHz to 2.4 GHz. Such RFID units are commercially available, for example from Phillips.

### Brief description of the figures

Figure 1a shows components of an RFID system.
Figure 1b shows the bulk reading of metallic objects in a 3-dimensional tunnel reader system.
Figure 1c shows maximum distances which still allow communication between a reader and RFID units differently placed on metallic objects.
Figures 2a and 2b show a tag with an RFID unit.
Figure 3 shows an aerosol container with a spacer.
Figure 4 shows an aerosol container with spacer and applied tag with RFID unit.

### Detailed description of the invention with reference to the figures

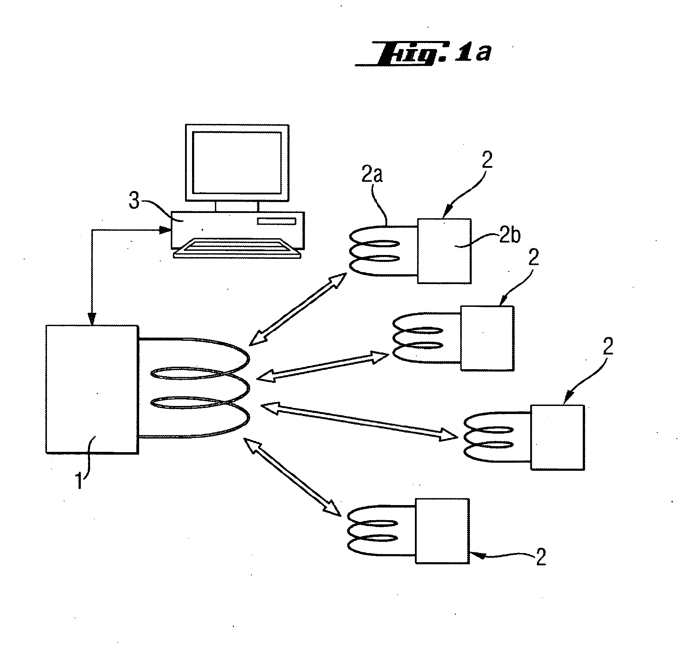

Figure 1a shows a schematic representation of the components of an RFID system. Data stored on RFID units (2) can be read out via a reader (1) and transferred to an electronic database (3). This can be stored on a PC (personal computer) or be part of an information management system.

The reader (1) transmits radiofrequency energy to the RFID unit or receives radiofrequency energy from there. The reader can, for example, be a long-range RFID reader with gate or tunnel antenna. The RFID units (2) are composed of an antenna (2a) connected to a microchip (2b) on which certain data can be stored, e.g. information concerning the identity of the aerosol container or concerning the use as test medication in clinical trials.

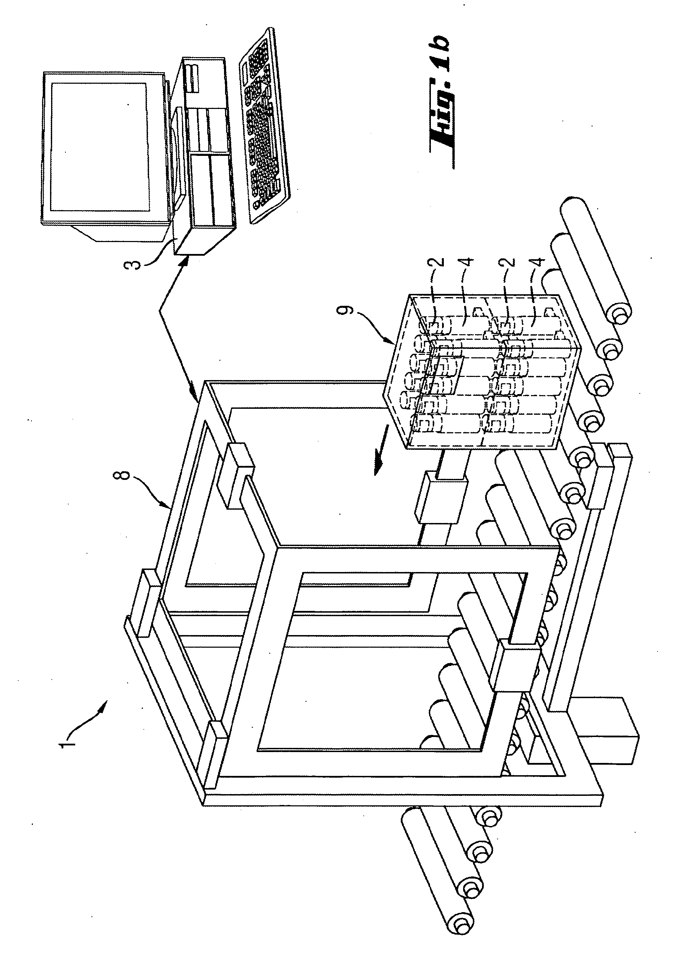

Figure 1b shows a reader arrangement with tunnel antenna (8). A packing (9) containing several aerosol containers (4) with RFID units (2) can be passed through this tunnel antenna for bulk reading.

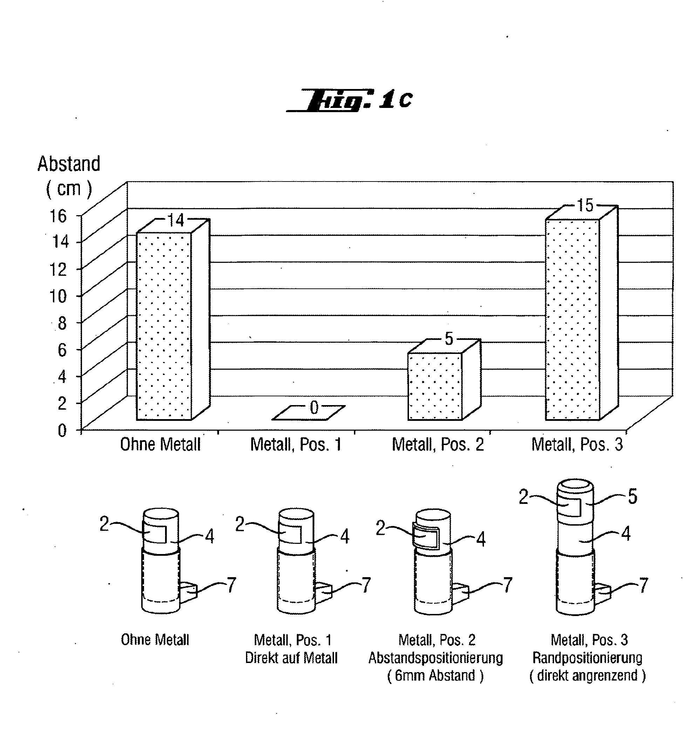

Figure 1c shows in comparison maximum distances which still allow communication between RFID units (2) placed on metallic aerosol container (4) and a reader system. As can be seen positioning the RFID unit on the outer circumference of the spacer (5) in parallel orientation to the metal surface of the aerosol container (4) allows for a maximum distance which still allows communication between the RFID unit (2) and a reader, comparable or even enhanced as placing the RFID unit (2) on a non-metal carrier. Directly positioning the RFID unit (2) on the metallic surface of the aerosol container (4) or placing the RFID unit (2) in about 6 mm distance (e.g. through an insulating spacer) on the metallic surface of the aerosol container (4) does not allow for reading the RFID unit or only allows for a minimal communication distance. Bulk reading in a 3-dimensional RFID tunnel reader for technical reasons requires communication taking place over a distance of at least between 10 to 15 cm (between reader antenna and RFID unit). Thus directly positioning the RFID unit (2) on the metal surface of the aerosol container (4) does not allow bulk reading in such a reader arrangement, while positioning the RFID unit (2) on the outer circumference of the spacer (5) does allow for bulk reading.

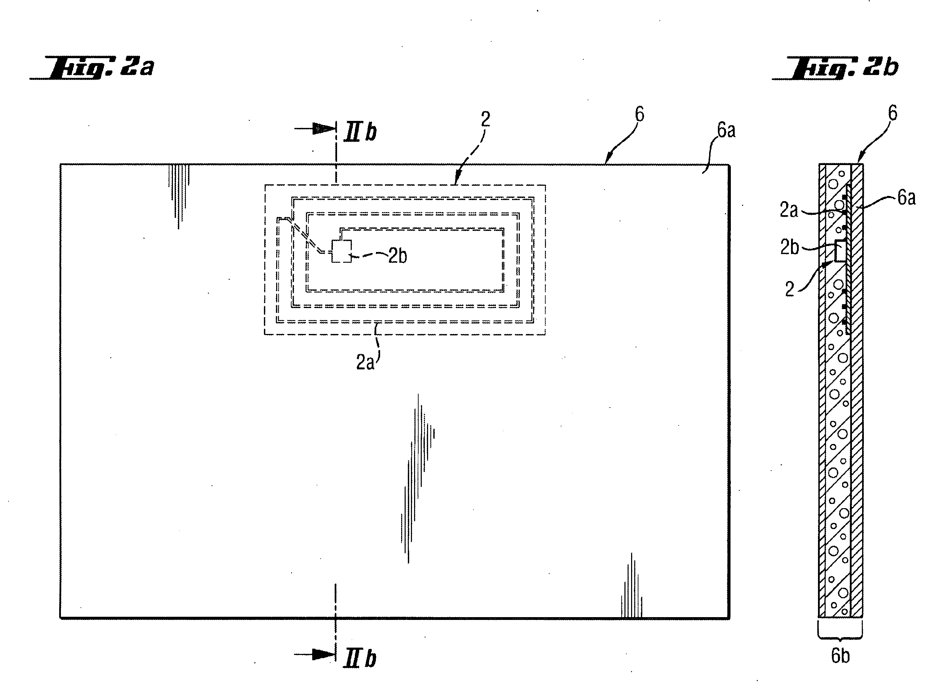

Figure 2 shows an RFID unit (2) which is connected to a tag (6). The tag is a tag with a self-adhesive layer: The tag is structured such that the RFID unit and the tag form one unit, the RFID unit being applied on the rear part of the tag, between film (6a) and adhesive layer (6b). The adhesive tag is dimensioned such that areas of the tag surround the spacer and the aerosol container and thus provide additional securing of the spacer on the aerosol container. At the same time, the useful surface area that can be printed on is increased. In one embodiment of the invention, the RFID unit (2) is positioned between the film layer (6a) and the adhesive layer (6b); to do this, the adhesive layer of the tag is lightly foamed (0.2 - 0.4 mm), so that the raised part of the RFID unit (2) can be pressed into the adhesive layer. This makes it possible to keep free as large as possible a tag surface area that can be printed on. This surface area can, if so desired, be printed with product information or other information. In this embodiment, the RFID unit (2) is a passive 13.56 MHz RFID unit (microchip connected to an antenna without energy source). An example that may be mentioned is the ISO Phillips semiconductor transponder with dimensions 32 x 16 mm. The latter can be read out with a reader (1) in the form of a 13.56 MHz long-range RFID reader with gate or tunnel antenna.

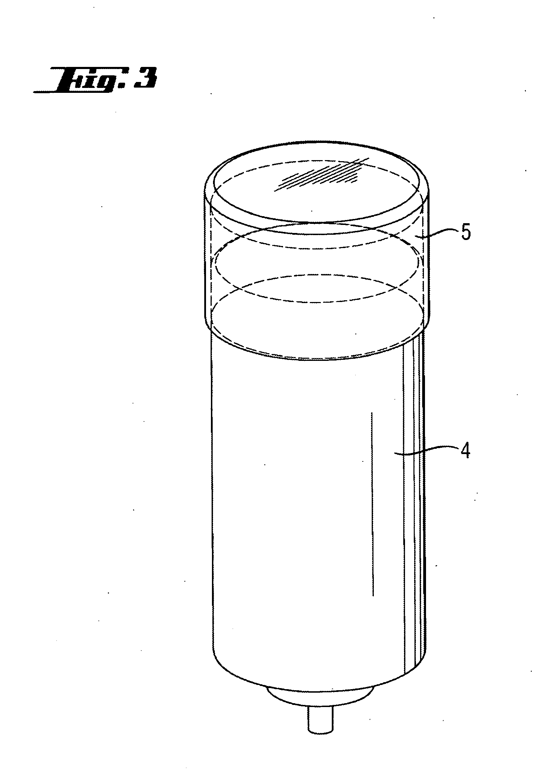

Figure 3 shows an aerosol container (4) with an applied spacer (5) in the form of a plastic cylinder. The plastic cylinder has the function of arranging the RFID unit away from the metal surface of the aerosol container (4) and thus permitting induction through the antenna. A blinding of the base of the aerosol container (4) is also effected. Spacer and aerosol container (4) are connected to one another preferably indivisibly as cap or by means of other securing means, for example a synthetic resin adhesive. If the connection is made using a synthetic resin adhesive, the latter can also at the same time compensate for small depressions. In the case of a plastic cap being fitted, this preferably has a slightly smaller internal diameter than the external diameter of the aerosol container, in order to obtain a suitable press fit.

The dimensions of the spacer (5) depend on the size of the aerosol container (4) to which it is to be applied. The cylinder according to the invention preferably has a height of 15 to 17 mm, preferably 16 mm, and a diameter of 23.2 mm. Moreover, the spacer (5) must be configured in such a way that, after the aerosol container has been fitted into a mouthpiece, the flow of air required for the inhalation process is not impaired. The RFID unit is preferably applied to the spacer such that there is no overlapping of the antenna of the transponder with the metal surface of the aerosol container. It is advantageous for the antenna of the transponder to be arranged in direct or close contact with the metal surface of the aerosol container, since a strengthening of the antenna action can in this way be achieved.

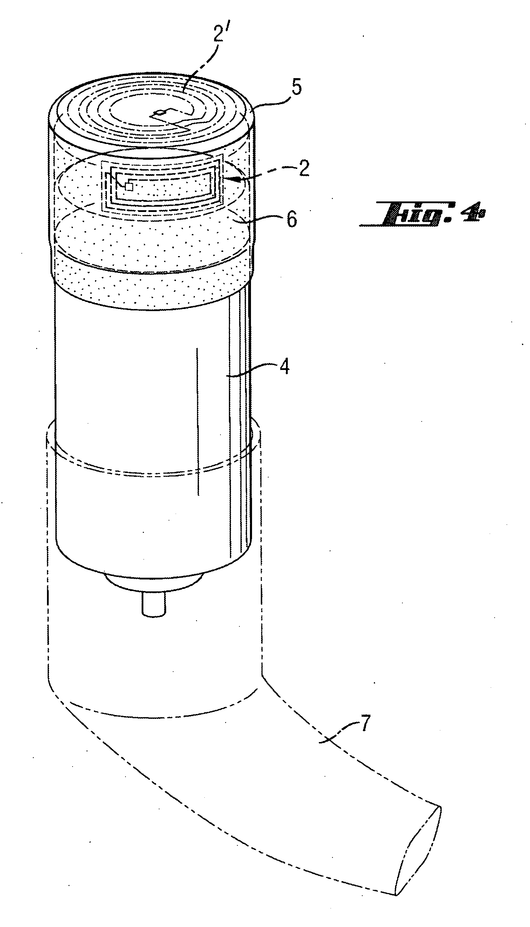

Figure 4 shows a metallic aerosol container (pressurized container) (4) according to the invention, with spacer (5) and applied tag (6) with RFID units (2), (2') on the outer circumference and, alternatively on the top surface. The mouthpiece (7) is shown schematically.

The aerosol container according to the invention is suitable, for example, for use in carrying out clinical trials.

## Claims

1. Metallic aerosol container (pressurized container) with a dispensing valve, in which a spacer made of an insulating material is applied to the base of the aerosol container, and an RFID unit is applied to the spacer.

2. Aerosol container according to Claim 1, wherein the spacer is a plastic cylinder.

3. Aerosol container according to Claim 1, in which the RFID unit is connected to a tag which is applied on the spacer by means of a self-adhesive layer.

4. Aerosol container according to Claim-3, wherein the adhesive tag is dimensioned such that areas of the tag surround the spacer and the aerosol container.

5. Aerosol container according to Claim 1, in which the RFID unit is applied to the outer circumference of the spacer.

6. Aerosol container according to Claim 5, wherein the RFID unit is in parallel orientation to the metal surface of the aerosol container.

7. Aerosol container according to Claim 5, in which the RFID unit has an antenna which is applied in direct or close contact with the metal surface of the aerosol container, so that a strengthening of the antenna action is achieved.

## Patentansprüche

1. Metallischer Aerosolbehälter (Druckbehälter) mit einem Abgabeventil, bei dem ein aus einem isolierenden Material bestehendes Abstandsglied auf die Basis des Aerosolbehälters aufgebracht wird und eine RFID-Einheit auf das Abstandsglied aufgebracht wird.

2. Aerosolbehälter nach Anspruch 1, wobei das Abstandsglied ein Kunststoffzylinder ist.

3. Aerosolbehälter nach Anspruch 1, wobei die RFID-Einheit mit einem Etikett verbunden ist, das mittels einer selbstklebenden Schicht auf dem Abstandsglied aufgebracht wird.

4. Aerosolbehälter nach Anspruch 3, wobei das klebende Etikett so bemessen ist, dass Bereiche des Etiketts das Abstandsglied und den Aerosolbehälter umgeben.

5. Aerosolbehälter nach Anspruch 1, bei dem die RFID-Einheit auf den äußeren Umfang des Abstandsglieds aufgebracht wird.

6. Aerosolbehälter nach Anspruch 5, wobei sich die RFID-Einheit in paralleler Orientierung zu der Metalloberfläche des Aerosolbehälters befindet.

7. Aerosolbehälter nach Anspruch 5, bei dem die RFID-Einheit eine Antenne aufweist, die in direktem oder engem Kontakt mit der Metalloberfläche des Aerosolbehälters aufgebracht ist, so dass eine Verstärkung der Antennenwirkung erzielt wird.

## Revendications

1. Contenant aérosol métallique (contenant sous pression) comprenant une valve de distribution, dans lequel un dispositif d'espacement fabriqué en matériau isolant est appliqué à la base du contenant aérosol, et une unité RFID est appliquée au dispositif d'espacement.

2. Contenant aérosol selon la revendication 1, dans lequel le dispositif d'espacement est un cylindre en plastique.

3. Contenant aérosol selon la revendication 1, dans lequel l'unité RFID est connectée à une étiquette qui est appliquée sur le dispositif d'espacement au moyen d'une couche auto-adhésive.

4. Contenant aérosol selon la revendication 3, dans lequel l'étiquette adhésive est dimensionnée de telle sorte que des zones de l'étiquette entourent le dispositif d'espacement et le contenant aérosol.

5. Contenant aérosol selon la revendication 1, dans lequel l'unité RFID est appliquée sur la circonférence extérieure du dispositif d'espacement.

6. Contenant aérosol selon la revendication 5, dans lequel l'unité RFID est orientée parallèlement à la surface métallique du contenant aérosol.

7. Contenant aérosol selon la revendication 5, dans lequel l'unité RFID a une antenne qui est appliquée en contact direct ou rapproché avec la surface métallique du contenant aérosol, de sorte qu'un renforcement de l'action de l'antenne soit obtenu.
